# EUROPEAN PATENT APPLICATION

(11) **EP 4 523 736 A1**
(43) Date of publication of application: **19.03.2025**
(21) Application number: 23197904.8
(22) Date of filing: 18.09.2023
(51) Int. Cl.: A61N 5/10, A61B 5/055

(54) **MAGNETIC RESONANCE IMAGING RADIATION THERAPY SYSTEM**

(71) Applicant: Koninklijke Philips N.V., 5656 AG Eindhoven (NL)
(72) Inventor: FORTHMANN, Peter, Eindhoven (NL); LIPS, Oliver, Eindhoven (NL); VERNICKEL, Peter, 5656AG Eindhoven (NL)
(74) Representative: Philips Intellectual Property & Standards

(57) **Abstract**

The invention relates to a magnetic resonance imaging radiation therapy system with reduced interference to the therapeutic radiation. The system includes: a magnetic resonance imager (110) comprising a magnet (120) enclosing a bore (130); a radiation therapy device (140) configured to radiate, from multiple positions about a longitudinal axis (150) of the bore, a therapeutic beam (160) with respective radial beam paths towards the bore (130); a coldhead (170) positioned outside of the beam paths; and a thermally conducting cooling segment (180) in thermal connection with the coldhead (170). The cooling segment (180) is extending azimuthally about the longitudinal axis (150) of the bore. The cooling segment (180) is positioned to intersect with the beam paths, and a width (w)of the cooling segment is larger than a maximum width of the therapeutic beam (180). Preferably, attenuation of the therapeutic beam (160) by the cooling segment (180) is constant between the respective beam paths from different positions about the longitudinal axis (150) of the bore.

## Description

### FIELD OF THE INVENTION

The invention relates to a magnetic resonance imaging radiation therapy system.

### BACKGROUND OF THE INVENTION

Magnetic resonance imaging (MRI) produces images for diagnosing disease and contrasting healthy tissue from abnormal tissue. An MRI system typically employs a superconducting magnet to generate the large magnetic fields which it requires for operation. To realize superconductivity, a magnet is maintained in a cryogenic environment at a temperature near absolute zero. Typically, the magnet includes one or more electrically (super-)conductive coils which are disposed in a cryostat and through which an electrical current circulates to create the magnetic field. Conduction cooling may form a promising alternative to cryostats with a bath of liquid Helium for cooling of systems with superconductors, such as MRI systems. As an example, Baig et. al, Supercond Sci Technol. 2017 April; 30(4), provides conceptual designs of 1.5 and 3.0 T full-body MRI magnets using conduction cooled MgB2 superconductor.

Radiation therapy can focus a radiation beam (radiotherapy beam) on a target region of interest in a patient and preferentially destroy diseased tissue while avoiding healthy tissue. The diagnostic spatial specificity of MRI can be combined with radiotherapy beam focus technology to provide more accurate treatment of diseased tissue while reducing the damage of healthy tissue. By combining real time imaging and radiation therapy, radiotherapy beam shaping may be performed in real time. This makes it possible to compensate for daily changes in anatomy, body movements such as breathing, which occur during the treatment procedure etc.

Generally, the radiation (e.g. X-ray) source can move around the magnet on the gantry of the MRI system and administer radiation to the patient through the cryostat from various positions. A radiotherapy beam may be attenuated when it passes through matter such as metals or liquid helium in a cryostat of the MRI system. In MRI linear accelerator (LINAC) systems or other MRI radiation therapy systems, the MRI cryostat for cooling of the system may therefore interfere with therapeutic radiation. Interference between the therapeutic radiation and the MRI cooling system may lead to unnecessary dose received by the patient, uneven radiation from different positions of the radiation source, scattered radiation etc.

### SUMMARY OF THE INVENTION

It is, inter alia, an object of the invention to provide an MRI radiation therapy system with reduced interference to the therapeutic radiation.

The invention is defined by the independent claims. Advantageous embodiments are defined in the dependent claims.

According to a first aspect, there is provided a magnetic resonance imaging radiation therapy system. The system comprises: a magnetic resonance imager comprising a magnet enclosing a bore; a radiation therapy device configured to radiate, from multiple positions about a longitudinal axis of the bore, a therapeutic beam with respective radial beam paths towards the bore; a coldhead positioned outside of the beam paths; and a thermally conducting cooling segment in thermal connection with the coldhead. The cooling segment is extending azimuthally about the longitudinal axis of the bore. The cooling segment is positioned to intersect with the beam paths, and a width of the cooling segment is larger than a maximum width of the therapeutic beam.

The magnetic resonance imager may also be referred to as an MRI system and is configured to generate magnetic resonance images of an object in the bore.

The coldhead functions as part of the `refrigerator', also known as `cryocooler', of the system. The cryocooler generally comprises a compressor to compress a working medium such as Helium. The compressor may be placed remote from the magnet. The coldhead is at the `cold end' of the cryocooler, which preferably uses a cooling mechanism of expansion and/or phase change of the working medium. The coldhead is positioned out of all the beam paths and will therefore not interfere with the therapeutic radiation. Preferably, the coldhead is positioned close to the coils to be cooled.

The cooling segment is thermally connected to the coldhead. The thermal connection may be via direct contact between the elements and/or via e.g. a heat conducting cooling connector. Such a cooling connector may be of the same material as the cooling segment or may be of another thermally conducting material. Cooling connectors may be e.g. heat pipes with working fluid, cooling straps, tubes, plates, braided structures etc. The cooling segment preferably comprises a metal, such as copper or aluminum. However, other heat conducting materials are also possible, such as e.g. carbon (nanotubes). The cooling segment may have the shape of a plate, sheet or similar bent in an arc or circle around the longitudinal axis of the bore. The cooling segment extending azimuthally about the longitudinal axis of the bore may have a curvature or otherwise non-flat shape in the direction of the longitudinal axis, such as e.g. the shape of a section of a sphere or ellipsoid. This may be advantageous for homogenous attenuation of a therapeutic beam that is e.g. slightly tilted with respect to the longitudinal axis of the bore. The cooling segment is positioned in the beam paths and has a width larger than the maximum width of the therapeutic beam. Since the coldhead is positioned outside the beam paths, any attenuation of the therapeutic beam by the cooling system may therefore come from the cooling segment only. Hence, interference to the radiation therapy from cooling straps, edges of the cooling segment, other connectors etc. is avoided while still allowing cooling along the length of the bore.

The radiation therapy device may be a linear accelerator, configured to deliver high energy X-ray or electron beams, a device for delivering particle therapy, or similar. The radiation therapy device may advantageously be configured to rotate about the longitudinal axis of the bore.

According to an embodiment of the invention, the cooling segment is configured such that attenuation of the therapeutic beam by the cooling segment is constant between the respective beam paths from different positions about the longitudinal axis of the bore. In this way, attenuation of the therapeutic beam is substantially constant along the entire length and width of the cooling segment, at least as far as the length and width are positioned in the beam paths. The cooling segment may have a constant thickness. Alternatively, the cooling segment may have a varying thickness combined with e.g. a varying density and/or material composition etc. to achieve constant attenuation. Since the attenuation is constant from the multiple positions, local interference to the radiation therapy beam may be reduced, accuracy of dose calculations and control may be improved etc.

According to an embodiment of the invention, the cooling segment has a radial thickness of less than 3 mm, preferably less than 2 mm and more preferably less than 1 mm. Due to the small thickness of the cooling segment, attenuation of the therapy beam is limited. The width of the cooling segment may be on the order of centimeters, such as more than 10 cm.

According to an embodiment of the invention, the cooling segment is a braided structure comprising thin braids. The braided structure provides heat conduction combined with mechanical flexibility, which may improve ease of assembly of the cooling segment without pre-bending. The material or materials of the cooling segment, braided patterns, etc. are chosen such that the braided structure does not cause unnecessary scattering of the therapeutic beam.

According to an embodiment of the invention, the magnet comprises a support structure for mechanically supporting the cooling segment. The support structure may be substantially transparent to radiation of the therapeutic beam. The support structure may hence provide negligible or low and preferably homogeneous attenuation for the therapeutic beam. Alternatively, or additionally, the support structure may be positioned outside of the beam paths. E.g., the support structure may be positioned along one or more of the edges of the cooling segment, like a flange. Support structures that are substantially transparent to the radiation of the therapeutic beam may comprise e.g. aluminum, glass, carbon, cotton fiber, glue, aerogel, paper, plastics, glass-fiber reinforced compositions etc. The support structure may be in the shape of a (partial) rim to support the cooling segment in an arc or circle. The support structure advantageously provides stability to the cooling segment but does not significantly interfere with the therapeutic beams. Thanks to the support structure, the cooling segment does not have to be freestanding, which may be beneficial for the thickness of the cooling segment, ease of manufacturing etc.

According to an embodiment of the invention, the magnet comprises a first magnet coil and a second magnet coil. The first magnet coil is positioned around the bore on a first longitudinal side of the cooling segment, and the second magnet coil is positioned around the bore on a second longitudinal side of the cooling segment. The coldhead is positioned on the first longitudinal side and thermally connected to the first magnet coil. The cooling segment is thermally connected to the second magnet coil on the second longitudinal side. Since the magnet coils are on either side of the cooling segment (along the longitudinal axis of the bore) the cooling segment is thermally connected to the second magnet coil on one side and to the coldhead on the other side. The first magnet coil is thermally connected to the coldhead on the first side and the second magnet coil is thermally connected to the cooling segment on the second side. In this way, each of the multiple magnet coils may be cooled with attenuation of the therapeutic beam coming only from the cooling segment.

According to another embodiment of the invention, the magnet comprises a first magnet coil and a second magnet coil. The first magnet coil is positioned around the bore on a first longitudinal side of the cooling segment, and the second magnet coil is positioned around the bore on a second longitudinal side of the cooling segment. The cooling segment is thermally connected to the first magnet coil on the first longitudinal side. The cooling segment is thermally connected to the second magnet coil on the second longitudinal side. This embodiment is similar to the one above. However, in this case total number of connections to the cold head may be limited. Furthermore, the design with coils and cooling segment may be symmetric, such that bill of material for manufacturing can be reduced.

According to an embodiment of the invention, the magnet comprises at least one cooling connector for providing thermal connection, and each of the at least one cooling connector is positioned outside of the beam paths of the therapeutic beam. Such cooling connectors may provide thermal connection between the coldhead and magnet coils, between the cooling segment and the coldhead, between the cooling segment and magnet coils, between magnet coils etc. Since none of the cooling connectors pass through the region where the cooling segment is positioned, i.e. none of the cooling connectors pass through any of the beam paths, interference with the therapeutic beam can be avoided. As mentioned above, the cooling connectors may be e.g. heat pipes with working fluid, cooling straps, tubes, plates, braided structures etc.

These and other aspects of the invention will be apparent from and elucidated with reference to the embodiments described hereinafter.

### BRIEF DESCRIPTION OF THE DRAWINGS

Fig. 1 schematically illustrates a cross section of a magnetic resonance imaging radiation therapy system according to embodiments of the invention.
Fig. 2 schematically illustrates a setup with multiple magnet coils according to embodiments of the invention.
Fig. 3 schematically illustrates a setup with multiple magnet coils according to embodiments of the invention.
Fig. 4 schematically illustrates a cooling segment and support structures according to embodiments of the invention.

### DETAILED DESCRIPTION OF EMBODIMENTS

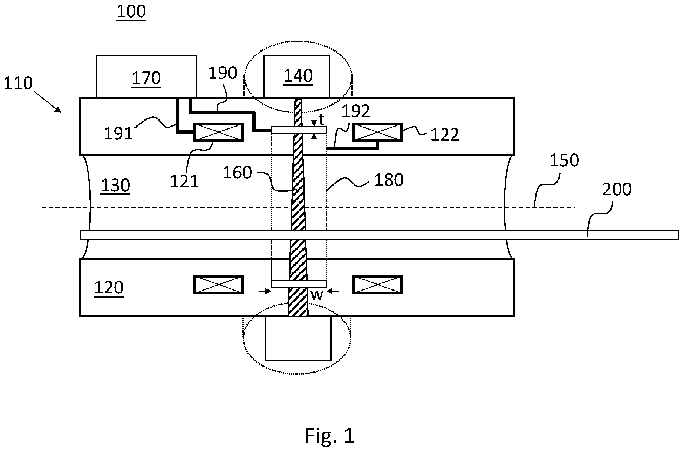

The schematic drawing in Fig. 1 illustrates an example of a cross section of a magnetic resonance imaging radiation therapy system 100. The system combines a magnetic resonance imager 110 with a radiation therapy device 140, such as a linear accelerator. The magnetic resonance imager 110 includes a magnet structure 120 that surrounds a bore 130. The magnet includes magnet coils 121, 122. A longitudinal axis 150 of the bore 130 is here illustrated with a dashed line. During imaging of an object, such as a patient, the object may be placed on a patient table 200 and positioned inside the bore 130. The radiation therapy device 140 may be used to provide radiation therapy to an object inside the bore 130 with a radiation therapy beam 160. In the system in Fig. 1, the radiation therapy device 140 can rotate fully or partly around the magnet 120 and hence around a section of the longitudinal axis 150 of the bore 130. In this way, the radiation therapy device 140 may provide radiation therapy beams from several radial angles to (part of) an object located inside the bore 130. In this example, the radiation therapy device provides therapeutic beams towards the central part of the bore, i.e., to the middle of the bore 130 along the longitudinal axis 150. However, skilled person will appreciate that many different design options with respect to e.g. positions and paths for the radiation therapy device 140 are possible in order to radiate therapeutic beams towards an object located in the bore 130. Conduction cooling of the magnet is provided by the coldhead 170. The coldhead 170 is placed outside paths of the radiation therapy beam 160. The coldhead 170 is in this example thermally connected directly to the first magnet coil 121 via a cooling connector 191. The coldhead is also connected to a cooling segment 180. The cooling segment 180 forms an arc or cirle around the (longitudinal axis 150) of the bore 130 and is positioned in the paths of the radiation therapy beam 160 from the different rotational positions of the radiation therapy device 140. The cooling segment 180 has a width w and a thickness t. The cooling segment 180 is preferably thin, such as with a thickness t of less than 3 mm, preferably less than 2 mm and more preferably less than 1 mm. The width w is larger than the width of the therapeutic beam 160. Furthermore, the position of the cooling segment 180 is preferably chosen such that the therapeutic beam 160 does not intersect with the edge of the cooling segment, which could cause scattering of the radiation. Preferably, attenuation of the therapeutic beam 160 by the cooling segment 180 is constant from all different positions of the radiation therapy device 140. In the example, the cooling segment is thermally connected to the second magnet coil 122 with a cooling connector 192. As seen from the figure, both magnet coils 121, 122 are cooled by the coldhead without any cooling connectors passing through the beam paths of the therapeutic beam 160. Therefore, attenuation of the therapeutic beam 160 by the cooling system may therefore come from the cooling segment 180 only. Hence, interference to the radiation therapy beam 160 can be avoided while still providing cooling of magnet coils 121, 122 on both sides (along the length of the bore) of the radiation therapy device 140.

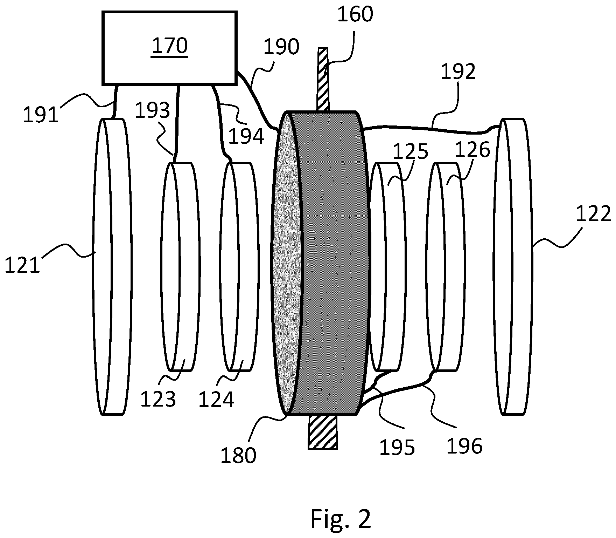

Fig. 2 schematically shows a setup with multiple magnet coils. In this case the coldhead 170 is thermally connected to each of the magnet coils 121, 123, 124 on the 'left' side of the radiation therapy beam paths via respective cooling connectors 191, 193, 194. The coldhead 170 is also thermally connected to the cooling segment 180 via a cooling connector 190. The coldhead 170, the magnet coils 121, 123, 124 and the cooling connectors 191, 193, 194 are positioned outside of paths of the radiation therapeutic beam 160. The cooling segment 180 is positioned in the paths of the radiation therapeutic beam 160. In the figure, only one path of the beam 160 is illustrated but it is to be understood that also other paths of the beam 160 from a radiation therapy device (not shown) also intersect with the cooling segment 180 from different rotational positions around the cooling segment 180. Additional magnet coils 122, 125, 126 are present at the `right' side of the radiation therapy beam paths. These magnet coils 122, 125, 126 are thermally connected to the cooling segment 180, and thus to the coldhead 170. The magnet coils 122, 125, 126 on the 'right' side are connected to the cooling segment via cooling connectors 192, 195, 196. Also in this case, the magnet coils 122, 125, 126 and the cooling connectors 192, 195, 196 are positioned outside the possible paths of the radiation therapeutic beam 160. Hence, the multiple magnet coils 121, 122,... 126 are conduction cooled by the coldhead 170 without causing disturbance to the paths of the radiation therapeutic beam 160.

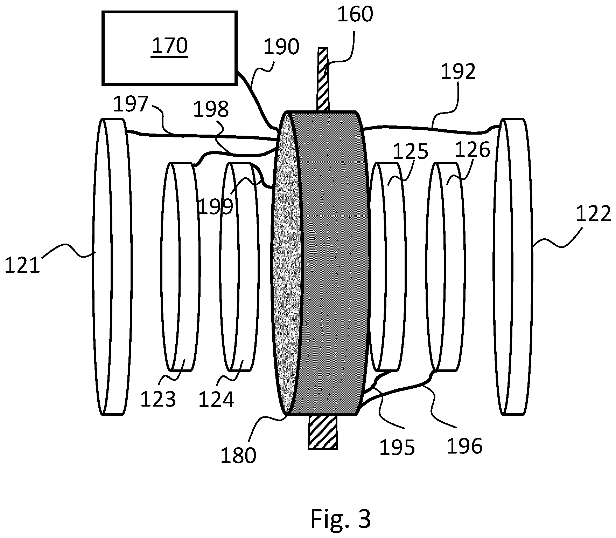

Fig. 3 schematically illustrates a setup that is similar to the one in Fig. 2. The difference is that in this case all magnet coils 121, 122,... 126 are thermally connected directly to the cooling segment 180 via cooling connectors 192, 195, 196,... 199. The cooling segment 180 is thermally connected to the coldhead 170, such that all the magnet coils can be cooled. Also in this example, neither the magnet coils, the cooling connectors nor the coldhead are interfering with the paths of the therapeutic beam 160.

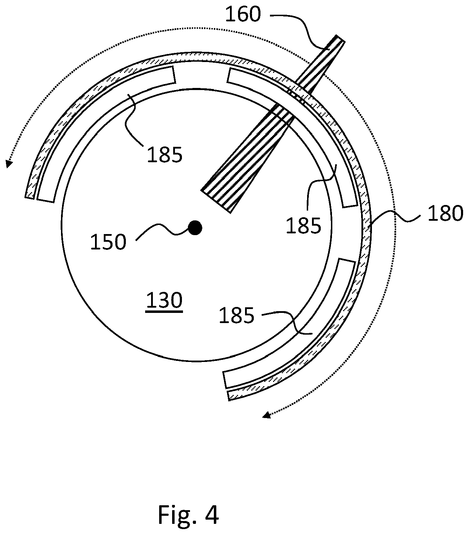

Fig. 4 schematically illustrates a cooling segment 180 and support structures 185, as viewed when looking into (or out of) the bore 130. I.e., from a view along the longitudinal axis 150 of the bore. In this example, the cooling segment 180 has the form of an arc. The therapeutic beam 160 may enter the bore 130 from different paths around the arc, as indicated with the dotted arrows. For each of the possible beam paths, the therapeutic beam 160 will pass through the cooling segment 180. The cooling segment 180 is supported by support structures 185. The support structures 185 provide support to the cooling segment 180 without significantly interfering with the therapeutic beam 160. The support structures may be substantially transparent to radiation of the therapeutic beam. Examples of support structure materials that may be used for this purpose include but are not limited to aluminum, glass, carbon, cotton fiber, glue, aerogel, paper, plastics, glass-fiber reinforced compositions etc. Alternatively, or additionally, the support structures may be placed outside of the beam paths, such as along the edges of the cooling segment. In the figure, three separate support structures 185 are shown. However, various options are conceivable, such as one larger support structure 185 or multiple smaller structures 185. Thanks to the support structures 185, the cooling segment may have relaxed design criteria with respect to mechanical stability, which may improve ease and cost of manufacturing, enable thinner structures etc.

It should be noted that the above-mentioned embodiments illustrate rather than limit the invention, and that those skilled in the art will be able to design many alternative embodiments without departing from the scope of the appended claims. As an example, the thermal connections in Figs. 1-3 are illustrated as cooling connectors, such as connectors in the form of single- or multi-layer pipes, straps, braids etc. However, other design options to transfer heat are also conceivable. Such as e.g. thermal connection via direct contact between structures, heat conducting plates, multiple connectors, etc. that are placed outside the beam paths of the therapeutic beam.

In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. In the device claim enumerating several means, several of these means may be embodied by one and the same item of hardware. Measures recited in mutually different dependent claims may advantageously be used in combination.

## Claims

1. A magnetic resonance imaging radiation therapy system (100) comprising;
- a magnetic resonance imager (110) comprising a magnet (120) enclosing a bore (130);
- a radiation therapy device (140) configured to radiate, from multiple positions about a longitudinal axis (150) of the bore, a therapeutic beam (160) with respective radial beam paths towards the bore;
- a coldhead (170) positioned outside of the beam paths; and
- a thermally conducting cooling segment (180) in thermal connection with the coldhead (170), wherein the cooling segment (180) is extending azimuthally about the longitudinal axis (150) of the bore, wherein the cooling segment (180) is positioned to intersect with the beam paths, and wherein a width (w) of the cooling segment (180) is larger than a maximum width of the therapeutic beam (160).

2. The system according to claim 1, wherein the cooling segment (180) is configured such that attenuation of the therapeutic beam (160) by the cooling segment (180) is constant between the respective beam paths from different positions about the longitudinal axis (150) of the bore.

3. The system according to claim 1 or 2, wherein the cooling segment (180) has a radial thickness (t) of less than 3 mm, preferably less than 2 mm and more preferably less than 1 mm.

4. The system according to claim 1 or 2 or 3, wherein the cooling segment (180) is a braided structure comprising thin braids.

5. The system according to any of the preceding claims, wherein the magnet (120) comprises a support structure (185) for mechanically supporting the cooling segment (180), and wherein the support structure (185) is substantially transparent to radiation of the therapeutic beam (160) and/or the support structure (185) is positioned outside of the beam paths.

6. The system according to any of the preceding claims, wherein the magnet (120) comprises a first magnet coil (121) and a second magnet coil (122), wherein the first magnet coil is positioned around the bore (130) on a first longitudinal side of the cooling segment (180), and the second magnet coil is positioned around the bore (130) on a second longitudinal side of the cooling segment (180), wherein the coldhead (170) is positioned on the first longitudinal side and thermally connected to the first magnet coil (121), and wherein the cooling segment (180) is thermally connected to the second magnet coil (122) on the second longitudinal side.

7. The system according to any of claims 1-5, wherein the magnet (120) comprises a first magnet coil (121) and a second magnet coil (122), wherein the first magnet coil is positioned around the bore (130) on a first longitudinal side of the cooling segment (180), and the second magnet coil is positioned around the bore (130) on a second longitudinal side of the cooling segment (180), wherein the cooling segment (180) is thermally connected to the first magnet coil (121) on the first longitudinal side, and wherein the cooling segment (180) is thermally connected to the second magnet coil (122) on the second longitudinal side.

8. The system according to any of the preceding claims, wherein the magnet comprises at least one cooling connector (190, 191, 192) for providing thermal connection, and wherein each of the at least one cooling connector (190, 191, 192) is positioned outside of the beam paths of the therapeutic beam (160).
